# EUROPEAN PATENT APPLICATION

(11) **EP 4 821 910 A1**
(43) Date of publication of application: **14.10.2026**
(21) Application number: 24902553.7
(22) Date of filing: 26.11.2024
(51) Int. Cl.: C07C 209/62, C07C 209/02, C07C 209/16, C07C 211/55, C07C 211/06, B01J 29/70

(54) **METHOD FOR PRODUCING DIPHENYLAMINE**

(30) Priority: 13.12.2023 CN 202311712987
(71) Applicant: China Petroleum & Chemical Corporation, Beijing 100728 (CN); Sinopec Dalian Research Institute of Petroleum and Petrochemicals Co., Ltd., Lushunkou District Dalian, Liaoning 116045 (CN)
(72) Inventor: ZHAO, Xiangyu, Dalian, Liaoning 116045 (CN); QI, Wenbo, Dalian, Liaoning 116045 (CN); AI, Fubin, Dalian, Liaoning 116045 (CN); LI, Haomeng, Dalian, Liaoning 116045 (CN); LI, Lanpeng, Dalian, Liaoning 116045 (CN)
(74) Representative: karo IP
(86) International application number: PCT/CN2024/134562
(87) International publication number: WO 2025/124131

(57) **Abstract**

The present invention belongs to the technical field of fine chemical engineering, and specifically relates to a method for producing diphenylamine. The method comprises: in the presence of an olefin, allowing aniline to come into contact with a catalyst, and performing a condensation reaction, wherein there are 2-4 carbon atoms in the olefin. By means of introducing an olefin into a reaction system, the method for producing diphenylamine provided in the present invention can effectively improve the conversion rate of aniline and the stability of the reaction, where the conversion rate of aniline can reach a maximum of 40.2 mol% while maintaining relatively high selectivity.

## Description

### CROSS-REFERENCE TO RELATED APPLICATIONS

The application claims the benefit of China patent application No. "202311712987.5", filed on December 13, 2023, the content of which is specifically and entirely incorporated herein by reference.

### TECHNICAL FIELD

The present invention belongs to the technical field of fine chemical engineering, and specifically relates to a method for producing diphenylamine.

### BACKGROUND ART

Diphenylamine is a widely used chemical product, and has important applications in rubber additives, dyes, explosive stabilizers, fruit preservatives, medicaments and other fields. Up to now, there are more than 20 methods for synthesizing diphenylamine, and the raw materials for production comprise aniline, phenol, cyclohexanone, N-cyclohexylaniline, dicyclohexyl and the like, and the catalysts are mainly aluminum chloride, boron trifluoride, activated alumina, zeolite molecular sieves, etc. Among these methods, the method of producing diphenylamine with aniline as the unique raw material has the best atomic economy and the strongest feasibility for large-scale production, and has been industrialized. The production process of synthesizing diphenylamine from aniline is divided into intermittent method, gas phase continuous method, and liquid phase continuous method, wherein the liquid phase continuous method is the most advanced production process at present. However, the aniline conversion rate in the production process is low, which seriously limits the production efficiency of the process of synthesizing diphenylamine from aniline, it is also the main problem encountered in the current industrial production process of diphenylamine.

CN1186802A discloses a process for continuous synthesis of diphenylamine from aniline in the presence of hydrogen, wherein the process uses the raw material refined gas (containing about 75% hydrogen gas and 25% nitrogen gas) of the synthetic ammonia plant as the hydrogen gas source, and forms a gas phase circulation system between the diphenylamine device and the synthetic ammonia compression section. The advantage of the process is that it effectively improves the aniline conversion rate in the diphenylamine production process and prolongs the service life of said catalyst on the basis of making full use of hydrogen gas resources. Although the hydrogenation effect is relatively obvious, the aniline conversion rate still cannot exceed 30%.

CN103044270A also discloses a method for continuously synthesizing diphenylamine by using phenylamine in the presence of hydrogen, and the purpose of improving the conversion rate of phenylamine is achieved by developing a hydrogen dissolving process. The key point of said process is contacting the phenylamine raw material with hydrogen gas before entering into a reactor, dissolving the hydrogen gas into the phenylamine raw material, and the phenylamine raw material with the dissolved hydrogen gas enters into a reactor used for continuously synthesizing diphenylamine from phenylamine, and carries out the reaction of synthesizing diphenylamine with phenylamine. The advantage of this method is that the efficiency of synthesizing diphenylamine from phenylamine is improved by dissolving hydrogen. However, the conversion rate of phenylamine cannot be effectively improved by dissolving hydrogen.

### SUMMARY OF THE INVENTION

The present invention aims to solve the technical problems of low aniline conversion rate and poor reaction stability in the prior art, and provides a method for producing diphenylamine. The method of the present invention has the characteristics of high aniline conversion rate and desirable reaction stability, and rosy industrial application prospects.

The invention provides a method for producing diphenylamine, the method comprises: contacting aniline with a catalyst and performing a condensation reaction in the presence of an olefin; wherein there are 2-4 carbon atoms in the olefin.

In the existing reaction of preparing diphenylamine by condensation of aniline, the condensation reaction of aniline is carried out in the presence of hydrogen gas and/or nitrogen gas. However, the inventors of the present invention have found in their research that although the aforementioned method can suppress the occurrence of side reactions to a certain extent and improve the selectivity of diphenylamine, it is difficult to promote the condensation reaction of aniline, and the conversion rate of aniline is still low. In addition, CN106631829A discloses a method of introducing isopropanol into the reaction system to improve the conversion rate of diphenylamine, but the conversion rate of aniline still cannot exceed 30%. At the same time, when isopropanol consumes ammonia produced by the condensation reaction of aniline, water will be generated, which may poison the catalyst, thereby being unfavorable for the long-term operation of the reaction. Moreover, the introduction of liquid phase isopropanol also increases the difficulty of operation.

The method for producing diphenylamine provided by the present invention can effectively improve the conversion rate of aniline and the stability of the reaction by introducing an olefin into the reaction system. Compared with the existing preparation method, the conversion rate of aniline can reach a maximum of 40.2 mol% while maintaining the same selectivity level.

### DESCRIPTION OF THE PREFERRED EMBODIMENT

The terminals and any value of the ranges disclosed herein are not limited to the precise ranges or values, such ranges or values shall be comprehended as comprising the values adjacent to the ranges or values. As for numerical ranges, the endpoint values of the various ranges, the endpoint values and the individual point values of the various ranges, and the individual point values may be combined with one another to produce one or more new numerical ranges, which should be deemed to have been specifically disclosed herein.

The present invention provides a method for producing diphenylamine, the method comprises: contacting aniline with a catalyst and performing a condensation reaction in the presence of an olefin; wherein there are 2-4 carbon atoms in the olefin.

The inventors of the present invention have found in their research that by introducing C₂-C₄ olefins into the reaction system, the conversion rate of aniline can be effectively improved, and the overall selectivity of the target product is high. In addition, compared with the method of introducing isopropanol to consume ammonia in the prior art, the method provided in the present invention will not generate water, it avoids poisoning of the catalyst, thereby improving the stability of the reaction.

According to the present invention, the olefin is at least one selected from the group consisting of ethylene, propylene, 1-butene, 2-butene and isobutene, preferably ethylene and/or propylene.

According to some particularly preferred embodiments of the present invention, the olefin is propylene, an use of the preferred embodiment is conducive to further improving the conversion rate of aniline and the stability of the reaction.

According to some preferred embodiments of the present invention, the molar ratio of olefin to aniline is 1: (1-5), such as 1: 1, 1: 1.5, 1: 2, 1: 2.5, 1: 3, 1: 3.5, 1: 4, 1: 4.5, 1: 5 and other specific but non-limiting ratios. Preferably, the molar ratio of olefin to aniline is 1:(1.5-3). The above preferred embodiments are helpful to further ensure the selectivity of diphenylamine in the reaction solution and reduce the occurrence of side reactions.

According to the present invention, preferably, the olefin is present in gaseous form during the condensation reaction. In the above preferred circumstance, the olefin gas can not only play a steam stripping role, take the ammonia generated by the aniline condensation reaction out of the reaction system, but also consume a small amount of the generated ammonia, which is beneficial to improving the conversion rate of aniline and the selectivity of diphenylamine.

According to some preferred embodiments of the present invention, the conditions of the condensation reaction comprise: a reaction temperature within the range of 130-220°C, more preferably within the range of 150-200°C, liquid hourly volume space velocity of aniline within the range of 0.01-1 h⁻¹, more preferably within the range of 0.05-0.5 h⁻¹, a reaction pressure within the range of 1-5MPa, more preferably within the range of 3-5MPa. The pressures involved in the invention are all gauge pressures. The reaction temperature of the existing liquid phase continuous method for preparing diphenylamine is usually above 300°C. Compared with the prior art, the method provided by the present invention can greatly reduce the reaction temperature of diphenylamine synthesis, and it has a relatively high aniline conversion rate and diphenylamine selectivity at a low temperature and a low hourly volume space velocity.

According to the present invention, the contact reaction can be carried out in any conventional reactor in the art. Preferably, the contact reaction is performed in a fixed bed reactor, which is filled with a catalyst.

The present invention does not particularly limit the feeding modes of olefin and aniline, those skilled in the art can adjust the feeding modes according to the actual condition and specific requirement of the reactor. For example, both olefin and aniline can be fed into the fixed bed reactor by means of top feed and pass through the catalyst bed; or both olefin and aniline can be fed into the fixed bed reactor by means of bottom feed and pass through the catalyst bed; or olefin may be fed into the fixed bed reactor by means of bottom feed, and aniline may be fed into the fixed bed reactor by means of top feed and passes through the catalyst bed.

In order to further improve the conversion rate of aniline, preferably, both olefin and aniline are fed into the fixed bed reactor by means of bottom feed and pass through the catalyst bed.

The present invention has no particular limitation on the type and composition of the catalyst, and any catalyst conventionally used in the art for preparing diphenylamine by condensation reaction of aniline can be applied in the present invention. The invention has no particular limitation on the source of said catalyst, and the catalyst may be commercially available or prepared with any known method in the art.

According to some preferred embodiments of the present invention, the catalyst comprises β zeolite and a metal component.

According to the present invention, preferably, the molar ratio of silica to alumina in said β zeolite is within the range of 20-100, more preferably within the range of 50-70.

Preferably, the β zeolite is Hβ zeolite.

The present invention has a wide selection range for the metal component. In order to further improve the conversion rate of aniline, preferably, the metal component is at least one selected from alkali metals, more preferably at least one elected from the group consisting of lithium, sodium and potassium.

According to some preferred embodiments of the present invention, based on the total mass of the catalyst, the content of said β zeolite is within the range of 50-90wt%, such as 50wt%, 55wt%, 60wt%, 65wt%, 70wt%, 75wt%, 80wt%, 85wt%, 90wt% or the specific but not limiting content, or a random value within the range consisting of any two numerical values, and the content of said metal component calculated in terms of element is within the range of 0.1-40wt%, for example, it may be 0.1wt%, 0.5wt%, 1wt%, 2wt%, 3wt%, 5wt%, 10wt%, 15wt%, 20wt%, 25wt%, 30wt%, 35wt%, 40wt% or the specific but not limiting content, or a random value within the range consisting of any two numerical values.

Further preferably, based on the total mass of said catalyst, the content of said β zeolite is within the range of 65-75wt%, and the content of said metal component calculated in terms of element is within the range of 1-30wt%. Under the preferable condition, it is beneficial to ensure that the conversion rate of the raw material aniline is higher than 30%, and under certain process conditions, the side reactions are suppressed to obtain more high value-added products.

In the invention, the content of metal components in the catalyst is measured by an inductively coupled plasma optical emission spectrometer (ICP-OES) method.

According to some preferred embodiments of the present invention, the catalyst further comprises a binder to obtain a catalyst molding. The invention has a wide selection range for the binder, and any binder that can be used for catalyst molding in the art may be applied in the present invention. Preferably, the binder is alumina and/or silica, more preferably alumina.

Preferably, the content of said β zeolite is within the range of 50-90wt%, more preferably within the range of 65-75wt%, the content of said alkali metal component calculated in terms of metal element is within the range of 0.1-40wt%, more preferably within the range of 1-30wt%, and the content of said binder is within the range of 0.5-49.9wt%, more preferably within the range of 5-30wt%, based on the total mass of said catalyst. Under the above preferred composition, it is beneficial to ensure that the conversion rate of the raw material aniline is higher than 30%, and under certain process conditions, the side reaction is suppressed to obtain more high value-added products.

The present invention has no particular limitation on the shape of the catalyst, those skilled in the art can select the size and morphology of the catalyst according to actual needs.

Preferably, the catalyst may be strip-shaped or spherical particles. When the catalyst is strip-shaped, the cross-section of said catalyst may be cylindrical, clover-shaped or four-leaf clover-shaped. The cross-sectional width of the strip-shaped particles is within the range of 0.5-3.0mm, preferably within the range of 1.0-2.0mm. When the catalyst has a spherical shape, the particle diameter is within the range of 0.5-5.0mm, preferably within the range of 1.0-3.0mm.

The invention has no particular limitation on the source of the catalyst, which is commercially available or prepared with any known method in the art. For example, the catalyst can be prepared with the method disclosed in CN200510047489.9. For example, the catalyst of the present invention can be prepared with the following method:
(1) Contacting the Hβ zeolite with a solution of a soluble compound of the metal component;
(2) Subjecting the mixture obtained in step (1) to solid-liquid separation, washing and drying;
(3) Fully kneading and molding and then drying and calcining the modified Hβ zeolite obtained in step (2), a binder and/or a binder precursor and a processing aid (e.g., an extrusion aid and a peptizing agent).

Preferably, the contact temperature of step (1) is within the range of 15-35°C, and the time is within the range of 0.5-2h. For example, the contact may be performed at room temperature.

Preferably, the concentration of the solution of the soluble compound of the metal component is within the range of 0.05-4mol/L.

The present invention has no particular limitation on the operation mode and conditions of the solid-liquid separation and washing, those skilled in the art can select them according to actual needs. The solid-liquid separation can be performed by filtering, for example. The present invention has no particular limitation on the number of washing times, and it can be adjusted according to the actual washing situation. The detergent used for washing may be water.

The present invention has no particular limitation on the drying conditions in step (2). Preferably, the drying temperature is within the range of 100-130°C and the drying time is within the range of 8-12h.

In the invention, the binder precursor refers to any substance that can be calcined to obtain the binder. For example, the precursor of alumina may be pseudo-boehmite and/or aluminum hydroxide, which are well known among those skilled in the art.

The invention has no particular limitation on the processing aid, which may be a conventional choice in the art. The invention has no particular limitation on the dosage of said processing aid, those skilled in the art can select the dosage according to actual needs. The extrusion aid, for example, may be sesbania powder, and the peptizing agent may be dilute nitric acid and/or citric acid, wherein the dilute nitric acid generally has a concentration within the range of 3-15 wt%.

The present invention has no particular limitation on the molding mode, those skilled in the art can select the molding mode according to actual needs, for example, the molding may be extrusion molding, tablet molding, ball rolling molding.

Preferably, the drying temperature in step (3) is within the range of 100-130°C and the drying time is within the range of 8-12h.

Preferably, the calcination temperature in step (3) is within the range of 450-550°C, and the calcination time is within the range of 6-8h.

Another aspect of the present invention provides a method for producing diphenylamine from aniline, the method comprises: subjecting aniline and a catalyst to a contact reaction in the presence of propylene;
The temperature of the contact reaction is within the range of 130-220°C, preferably within the range of 150-200°C, the liquid hourly volume space velocity of aniline is within the range of 0.01-1 h⁻¹, preferably within the range of 0.05-0.5 h⁻¹, and the reaction pressure is within the range of 1-5MPa, preferably within the range of 3-5MPa.

The present invention will be described in detail below with reference to examples.

Unless otherwise specified in the present invention, the raw materials used in the following examples and comparative examples were commercially available.

In the following examples and comparative examples, the calculation methods of the conversion rate of aniline and the selectivity of diphenylamine were as follows:
The molar conversion rate of aniline:
   $ω = \frac{{n}_{0} - {n}_{2}}{{n}_{0}} \times 100 %$
Wherein *ω* denoted the molar conversion rate of aniline, mol%; n₀ denoted the mole number of aniline in the raw materials; n₂ denoted the mole number of aniline in the reaction solution.

The molar selectivity of diphenylamine:
${ε}_{1} = \frac{{n}_{3}}{{n}_{1}} \times 100 %$
Wherein *ε*₁ denoted the molar selectivity of diphenylamine, mol%; n₁ denoted the mole number of each product component in the reaction solution, mol; n₃ denoted the mole number of diphenylamine in the reaction solution, mol.

The reaction solution referred to the mixed liquid phase obtained after the reaction. The "product" in the above formulae did not include aniline. The composition of the reaction liquid was analyzed with reference to the Chemical Industry Standard HG/T5534-2019 in China.

The catalysts used in the examples and comparative examples were prepared with the following method:
(1) Contacting Hβ zeolite with a sodium chloride solution;
(2) Filtering, washing and drying the mixture obtained in step (1);
(3) Fully kneading and extrusion molding the modified Hβ zeolite obtained in step (2), the alumina binder precursor, the extrusion aid and the peptizing agent to obtain a strip-shaped catalyst with a diameter of 2mm and a length of 2-3mm, and then drying and calcining the strip-shaped catalyst.

Wherein the contact temperature of step (1) was 20°C and the contact time was 1.5 hours. The concentration of the sodium chloride solution was 0.5mol/L. Step (2) related to drying at 120°C for 10 hours, and step (3) involved with drying at 120°C for 10 hours and calcining at 500°C for 6 hours. The binder in use was aluminum hydroxide, and the zeolite in use was commercially available Hβ zeolite, which had a specific surface area of 650m²/g and a particle diameter of 1.5-2mm.

The properties of the catalysts were listed in Table 1.

**Table 1**

| Catalysts | | A | B | C | D |
|---|---|---|---|---|---|
| Metal Components | Types | Na | Na | Na | Na |
| | Content (wt%) | 2 | 5 | 20 | 0.5 |
| Hβ zeolite (wt%) | | 72 | 72 | 65 | 65 |
| Molar ratio of silica to alumina in Hβ zeolite | | 50 | 50 | 60 | 60 |
| Alumina | | Balance | Balance | Balance | Balance |

### Example 1

In a stainless steel fixed bed reactor with an inner diameter of 20mm and a length of 1,000mm, the filling amount of catalyst was 100mL. The reaction raw materials aniline and propylene (commercially available, with a purity larger than 99%) were introduced into the reactor by means of bottom feed. After the reaction product came out from the top of the reactor, it was cooled and then entered a separator. The reaction was carried out for 48 hours under the reaction conditions in Table 2. The composition of the reaction product was analyzed by gas chromatography, and the measured product distribution was shown in Table 2 below.

### Examples 2-7

The method for producing diphenylamine was performed according to the method in Example 1, except that the feed modes and the reaction conditions were as shown in Table 2 respectively, after the reaction was carried out for 48 hours, the composition of the reaction product was analyzed by gas chromatography, and the measured product distribution was shown in Table 2 below.

### Examples 8-11

The method for producing diphenylamine was performed according to the method in Example 1, except that the feed modes and the reaction conditions were as shown in Table 3 respectively, after the reaction was carried out for 48 hours, the composition of the reaction product was analyzed by gas chromatography, and the measured product distribution was shown in Table 3 below.

### Example 12

The method for producing diphenylamine was performed according to the method in Example 1, except that propylene was replaced with an equimolar amount of ethylene.

The composition of the reaction product was analyzed by gas chromatography, and the measured product distribution was shown in Table 3 below.

### Comparative Example 1

The method for producing diphenylamine was performed according to the method in Example 1, except that propylene was replaced with an equimolar amount of hydrogen gas. The specific reaction conditions and results were shown in Table 3.

### Comparative Example 2

Diphenylamine was synthesized according to the method of CN106631829A, wherein the molar ratio of isopropanol to aniline was 1: 1. The specific reaction conditions and results were as shown in Table 3.

**Table 2**

| | Examples | | | | | | |
|---|---|---|---|---|---|---|---|
| | 1 | 2 | 3 | 4 | 5 | 6 | 7 |
| Catalysts | A | B | C | B | D | B | B |
| Olefins | Propylene | Propylene | Propylene | Propylene | Propylene | Propylene | Propylene |
| Molar ratio of olefin: aniline | 1:3 | 1:2.5 | 1:1.5 | 1:2 | 1:2 | 1:0.5 | 1:4 |
| Feeding mode | Bottom feed | Bottom feed | Bottom feed | Bottom feed | Bottom feed | Bottom feed | Bottom feed |
| Reaction temperature, °C | 150 | 150 | 200 | 200 | 200 | 200 | 200 |
| Reaction pressure, MPa | 3.0 | 3.5 | 4.5 | 5.0 | 5.0 | 5.0 | 5.0 |
| Reaction time, h | 48 | 48 | 48 | 48 | 48 | 48 | 48 |
| Aniline liquid hourly volume space velocity, h⁻¹ | 0.05 | 0.1 | 0.4 | 0.5 | 0.5 | 0.5 | 0.5 |
| Aniline conversion rate (mol%) | 36.5 | 37.1 | 40.2 | 38.4 | 35.6 | 36.2 | 34.8 |
| Diphenylamine selectivity (mol%) | 96.8 | 97.2 | 96.9 | 97.3 | 95.8 | 95.0 | 96.5 |

**Table 3**

| | Examples | | | | | Comparative Examples | |
|---|---|---|---|---|---|---|---|
| | 8 | 9 | 10 | 11 | 12 | 1 | 2 |
| Catalysts | B | A | C | C | A | A | A |
| Olefins | Propylene | Propylene | Propylene | Propylene | Ethylene | / | / |
| Molar ratio of olefin: aniline | 1:2 | 1:3 | 1:1.5 | 1:1.5 | 1:3 | / | / |
| Feeding mode | Top feed | Bottom feed | Bottom feed | Bottom feed | Bottom feed | Bottom feed | Bottom feed |
| Reaction temperature, °C | 200 | 130 | 210 | 200 | 150 | 150 | 150 |
| Reaction pressure, MPa | 5.0 | 3.0 | 4.5 | 4.5 | 3.0 | 3.0 | 3.0 |
| Reaction time, h | 48 | 48 | 48 | 48 | 48 | 48 | 48 |
| Aniline liquid hourly volume space velocity, h⁻¹ | 0.5 | 0.05 | 0.4 | 0.8 | 0.05 | 0.05 | 0.05 |
| Aniline conversion rate (mol%) | 35.1 | 35.2 | 42.4 | 39.4 | 35.4 | 18.3 | 10.8 |
| Diphenylamine selectivity (mol%) | 96.2 | 92.8 | 82.6 | 88.3 | 96.3 | 78.5 | 56.6 |

### Examples 13-14

In a stainless steel fixed bed reactor with an inner diameter of 20mm and a length of 1,000mm, the filling amount of catalyst was 100mL. The reaction raw materials aniline and propylene (commercially available, with a purity larger than 99%) were introduced into the reactor by means of bottom feed. After the reaction product came out from the top of the reactor, it was cooled and then entered a separator. The reaction conditions and the product distribution were as shown in Table 4 below.

### Comparative Example 3

The method for producing diphenylamine was performed according to the method in Example 12, except that propylene was replaced with an equimolar amount of hydrogen gas.

The specific reaction conditions and results were as shown in Table 4 below.

### Comparative Example 4

Diphenylamine was synthesized according to the method of CN106631829A, wherein the molar ratio of isopropanol to aniline was 1: 1. The specific reaction conditions and results were as shown in Table 4 below.

**Table 4**

| | Example 13 | Example 14 | Comparative Example 3 | Comparative Example 4 |
|---|---|---|---|---|
| Catalysts | A | A | A | A |
| Olefins | Propylene | Propylene | / | / |
| Molar ratio of olefin: aniline | 1:2 | 1:4 | / | / |
| Reaction temperature, °C | 200 | 200 | 200 | 200 |
| Reaction pressure, MPa | 5.0 | 5.0 | 5.0 | 5.0 |
| Reaction time, h | 500 | 500 | 500 | 500 |
| Aniline liquid hourly volume space velocity, h⁻¹ | 0.5 | 0.5 | 0.5 | 0.5 |
| Aniline conversion rate (mol%) | 36.4 | 35.2 | 19.5 | 14.6 |
| Diphenylamine selectivity (mol%) | 96.5 | 95.8 | 80.3 | 52.5 |

As can be seen from the results of the above Examples and Comparative Examples, by means of introducing an olefin into a reaction system, the method for producing diphenylamine provided in the present invention can effectively improve the conversion rate of aniline and the stability of the reaction, the conversion rate of aniline can reach a maximum of 40.2 mol% while maintaining relatively high selectivity.

The above content describes in detail the preferred embodiments of the present invention, but the present invention is not limited thereto. A variety of simple modifications can be made in regard to the technical solutions of the present invention within the scope of the technical concept of the present invention, including a combination of individual technical features in any other suitable manner, such simple modifications and combinations thereof shall also be regarded as the content disclosed by the present invention, each of them falls into the protection scope of the present invention.

## Claims

1. A method for producing diphenylamine, the method comprises:
contacting aniline with a catalyst and performing a condensation reaction in the presence of an olefin;
wherein there are 2-4 carbon atoms in the olefin.

2. The method according to claim 1, wherein the olefin is at least one selected from the group consisting of ethylene, propylene, 1-butene, 2-butene and isobutene, preferably ethylene and/or propylene, more preferably propylene.

3. The method according to claim 1 or 2, wherein the molar ratio of olefin to aniline is 1: (1-5), preferably 1: (1.5-3).

4. The method according to any one of claims 1-3, wherein the olefin is present in gaseous form during the condensation reaction;
preferably, the conditions of the condensation reaction comprise: a reaction temperature within the range of 130-220°C, more preferably within the range of 150-200°C, liquid hourly volume space velocity of aniline within the range of 0.01-1 h⁻¹, more preferably within the range of 0.05-0.5 h⁻¹, a reaction pressure within the range of 1-5MPa, more preferably within the range of 3-5MPa.

5. The method according to any one of claims 1-4, wherein the contact reaction is performed in a fixed bed reactor.

6. The method according to claim 5, wherein both olefin and aniline are fed into the fixed bed reactor by means of top feed and pass through the catalyst bed; or
both olefin and aniline are fed into the fixed bed reactor by means of bottom feed and pass through the catalyst bed; or
olefin is fed into the fixed bed reactor by means of bottom feed, and aniline is fed into the fixed bed reactor by means of top feed and passes through the catalyst bed.

7. The method according to any one of claims 1-6, wherein the catalyst comprises β zeolite and a metal component.

8. The method according to claim 7, wherein the molar ratio of silica to alumina in said β zeolite is within the range of 20-100;
preferably, the β zeolite is Hβ zeolite;
preferably, the metal component is at least one selected from alkali metals, more preferably at least one elected from the group consisting of lithium, sodium and potassium.

9. The method according to claim 7 or 8, wherein the content of said β zeolite is within the range of 50-90wt%, and the content of said metal component calculated in terms of element is within the range of 0.1-40wt%, based on the total mass of the catalyst;
preferably, the content of said β zeolite is within the range of 65-75wt%, and the content of said metal component calculated in terms of element is within the range of 1-30wt%, based on the total mass of said catalyst.

10. The method according to any one of claims 7-9, wherein the catalyst further comprises a binder, the binder is preferably alumina and/or silica, more preferably alumina;
preferably, the content of said β zeolite is within the range of 50-90wt%, more preferably within the range of 65-75wt%, the content of said alkali metal component calculated in terms of metal element is within the range of 0.1-40wt%, more preferably within the range of 1-30wt%, and the content of said binder is within the range of 0.5-49.9wt%, more preferably within the range of 5-30wt%, based on the total mass of said catalyst.
